# DEMANDE DE BREVET EUROPEEN

(11) **EP 0 623 362 A1**
(43) Date de publication de la demande: **09.11.1994**
(21) Numéro de dépôt: 94201158.6
(22) Date de dépôt: 27.04.1994
(51) Int. Cl.: A61N 1/36, A61N 1/32

(54) **Appareil générateur de courant pour traitements thérapeutiques dans le domaine de la biophysique et de la physiologie**

(30) Priorité: 04.05.1993 FR 9305421
(71) Demandeur: L'ESPACE MEDICAL - LA MAISON DU MEDECIN, Société anonyme, F-31100 Toulouse (FR)
(72) Inventeur: Hamard, Claude, F-31450 Ayguesvives (FR); Mario, Bernard, F-82700 Montech (FR); Petitjeans, Robert, F-31100 Toulouse (FR)
(74) Mandataire: Cabinet BARRE LAFORGUE & associés

(57) **Abrégé**

L'invention concerne un appareil générateur de courant pour l'alimentation d'au moins deux sondes destinées à être appliquées sur une partie du corps humain en vue d'un traitement thérapeutique dans le domaine de la biophysique et de la physiologie. Cet appareil comprend une source (2) d'alimentation électrique autonome apte à délivrer une tension constante, des moyens (18) de génération d'un courant constant, des moyens (10, 19) générateurs de fréquence aptes à transformer le courant constant de façon à délivrer un courant présentant la forme d'impulsions rectangulaires biphasiques à moyenne nulle et fronts raides, dont la durée totale T en µs est sensiblement comprise entre 200 µs et 800 µs, et des moyens de temporisation (12, 14, 15) de l'envoi des départs d'impulsions aptes à déclencher une impulsion toutes les k x 15 ms, avec k = 1 ou 4.

## Description

L'invention concerne un appareil générateur de courant doté d'au moins une sortie de connexion pour l'alimentation d'au moins deux sondes destinées à être appliquées sur une partie du corps humain en vue d'un traitement thérapeutique dans le domaine de la biophysique et de la physiologie.

Les appareils générateurs de courant actuellement utilisés en vue de traitements thérapeutiques, et tels que notamment décrits dans les brevets EP 459.945 et US 4.327.322, émettent des stimuli en fréquences entières qui ont peu de corrélation avec la norme biologique. L'inconvénient de tels appareils réside dans le fait que l'obtention d'un résultat correct coïncide avec une énorme déperdition d'énergie, consistant en un grand pourcentage d'énergie rentrant dans le biologique sans être utilisable ou utilisée.

La présente invention vise à pallier cet inconvénient et a pour principal objectif de fournir un appareil générateur de courant dont les performances sont notablement optimisées par rapport à celles des appareils connus.

A cet effet, l'invention vise un appareil comprenant :
- une source d'alimentation électrique autonome apte à délivrer une tension constante,
- des moyens de génération d'un courant constant,
- des moyens générateurs de fréquence aptes à transformer le courant constant de façon à délivrer un courant présentant la forme d'impulsions rectangulaires biphasiques à moyenne nulle et fronts raides, dont la durée totale T en µs est sensiblement comprise entre 200 µs et 800 µs.

Selon l'invention, cet appareil se caractérise en ce qu'il comprend des moyens de temporisation de l'envoi des départs d'impulsions aptes à déclencher une impulsion toutes les k x 15 ms, avec k = 1 ou 4.

L'objet de l'invention a donc été de réaliser un appareil générateur de courant doté de moyens de temporisation conçus pour contrôler le temps s'écoulant entre deux départs d'impulsions, et pour déclencher une impulsion toutes les k x 15 ms avec k = 1 ou k = 4.

Autrement dit, l'idée a la base de l'invention a été de raisonner en termes de temporisation des départs d'impulsions, avec des valeurs très spécifiques (15 ms ou 60 ms) de cette temporisation, en faisant abstraction de toute référence à des notions de fréquence (exprimée en Hertz) desdites impulsions.

Une telle conception conduit à l'obtention d'un appareil générant des stimuli qui sont en totale corrélation avec la norme biologique, et permettant donc d'obtenir des résultats optimaux quant à la quantité d'énergie utilisable et utilisée lors d'un traitement thérapeutique.

Cet appareil offre, en outre, deux possibilités de calage des départs d'impulsions qui peuvent être déclenchées soit toutes les 15 ms, soit toutes les 60 ms.

A titre d'exemple, la périodicité de 60 ms peut être utilisée lors d'un traitement visant à aider le recrutement musculaire d'un muscle dont l'aptitude au recrutement est atteinte. Elle peut également être utilisée lors d'un traitement visant à renforcer le processus proprioceptif.

La périodicité de 15 ms permet quant à elle d'obtenir le rendement maximal possible des contractions du muscle, et peut par exemple être utilisée lors d'un traitement visant à obtenir un renforcement musculaire.

Cette périodicité de 15 ms conduisant à la tétanisation du muscle traité, l'appareil selon l'invention est conçu pour entrecouper le traitement de cycles de repos.

A cet effet, les moyens de temporisation comprennent avantageusement :
- un oscillateur de base doté d'un commutateur apte à permettre de sélectionner la périodicité, 15 ms ou 60 ms, des impulsions,
- un oscillateur de rapport de cycle agencé pour être actif lorsque la périodicité des impulsions est de 15 ms, et adapté pour scinder, de façon périodique, l'émission des impulsions en deux cycles de durées prédéterminées : un cycle de travail durant lequel les impulsions sont délivrées toutes les 15 ms, et un cycle de repos durant lequel aucune impulsion n'est délivrée.

En outre, selon un mode de réalisation préférentiel, cet oscillateur de rapport de cycle est adapté pour que le rapport entre la durée du cycle de repos et la durée du cycle de travail soit de trois.

De plus, l'appareil selon l'invention est également conçu pour limiter automatiquement la durée du traitement lorsque la périodicité des impulsions est de 15 ms. A cet effet, les moyens de temporisation comprennent avantageusement une horloge apte à interrompre l'émission des impulsions au bout d'un laps de temps prédéterminé après le début du traitement, lorsque la périodicité des impulsions est de 15 ms.

Selon une autre caractéristique de l'invention, les moyens générateurs de fréquence sont adaptés pour délivrer des impulsions dont la durée totale T peut être soit 300 µs, soit 600 µs, et comprennent un commutateur de sélection de l'une ou l'autre durée.

Ces deux valeurs s'avèrent, en effet, constituer le choix thérapeutique optimal, la sélection de l'une ou l'autre valeur permettant en outre de répondre à la chronaxie de chaque muscle.

En outre, selon une autre caractéristique de l'invention, les moyens générateurs de fréquence comprennent :
- un générateur d'impulsions apte à délivrer des impulsions positives d'une durée totale de T_{/2}, décalées l'une par rapport à l'autre de T_{/2},
- au moins un transformateur doté de deux bobinages primaires inversés possédant un point commun relié à un générateur de courant, lesdits bobinages primaires étant activés par le générateur d'impulsions de façon à générer le courant rectangulaire biphasique sur le secondaire du transformateur.

L'appareil selon l'invention comporte, en outre, avantageusement des moyens de réglage manuel de l'intensité du courant délivré vers les sorties de connexion, adaptés pour permettre de faire varier ladite intensité entre une valeur minimale nulle et une valeur prédéterminée.

Cet appareil est par ailleurs doté de modules de sécurité visant à surveiller l'intensité du courant délivré.

Le premier de ces modules de sécurité consiste en des moyens de détection de la position des moyens de réglage de l'intensité du courant, aptes à interdire l'émission d'impulsions lorsque, lors de la mise en marche de l'appareil, lesdits moyens de réglage sont dans une position différente de celle correspondant à la valeur d'intensité nulle.

De tels moyens de détection ont pour avantage d'assurer, lors de la mise en marche de l'appareil, une initialisation par une valeur d'intensité nulle.

Un second module de sécurité comprend, quant à lui, des moyens, dits de détection de changement de programme, aptes à interrompre l'émission d'impulsions lors de l'actionnement du commutateur de l'oscillateur de base ou des moyens générateurs de fréquence, alors que les moyens de réglage sont dans une position différente de celle correspondant à la valeur d'intensité nulle.

Enfin, des moyens de détection de défauts, tels que surtension, sonde déconnectée... sont aptes à interdire l'émission d'impulsions lors de l'apparition d'un tel défaut.

D'autres caractéristiques, buts et avantages de l'invention ressortiront de la description détaillée qui suit en référence aux dessins annexés qui en représentent à titre d'exemple non limitatif un mode de réalisation préférentiel. Sur ces dessins qui font partie intégrante de la présente description :
- la figure 1 est un schéma synoptique d'un appareil générateur de courant conforme à l'invention,

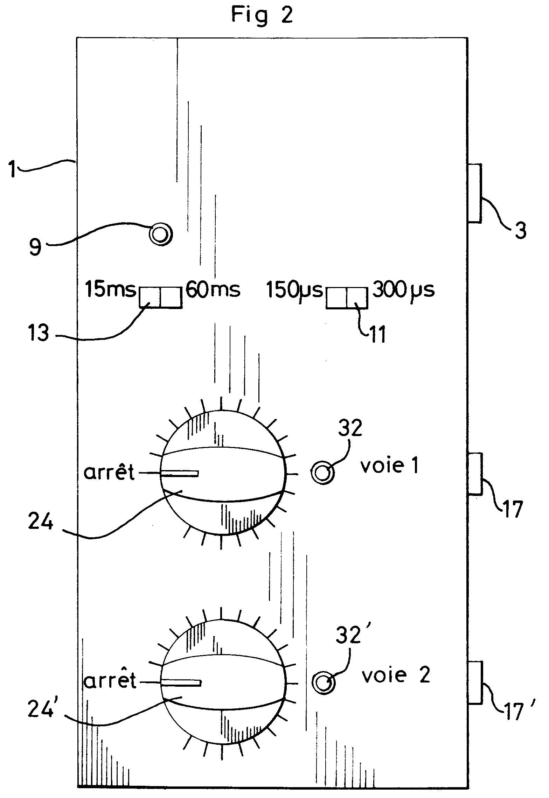

- la figure 2 est une vue de la face frontale du boîtier de cet appareil,
- et les figures 3a à 3d représentent les différentes formes de signaux générés par cet appareil.

L'appareil générateur de courant selon l'invention constitue un équipement médical de soins physiothérapiques (stimulateur musculaire). Cet appareil se présente sous la forme d'un boîtier portable 1 de dimensions suffisamment réduites (de l'ordre de 100 mm x 200 mm x 50 mm) pour pouvoir être tenu d'une main, avec possibilité de port à la ceinture en situation de marche.

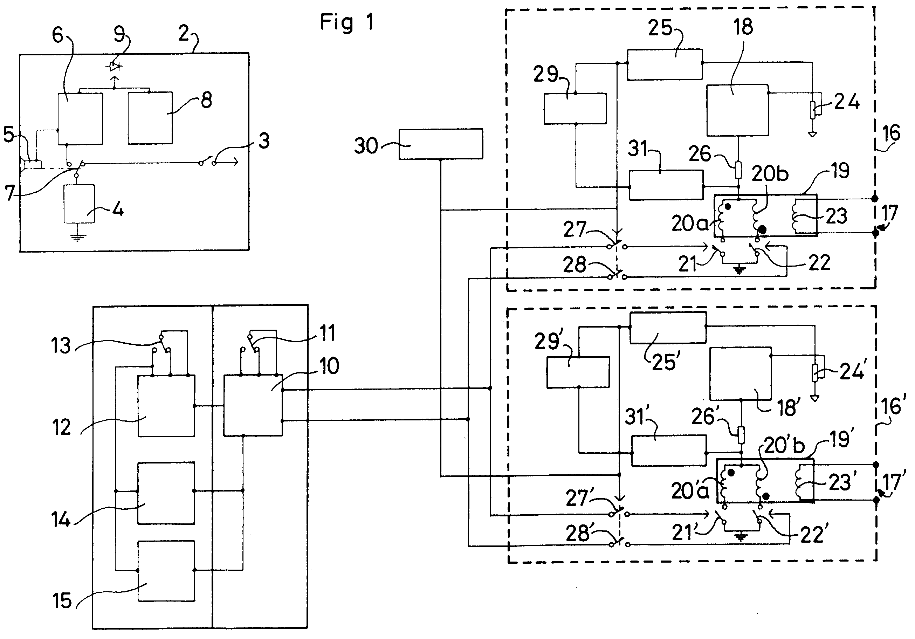

Tel que représenté à la figure 1, cet appareil comprend, en premier lieu, une source d'alimentation électrique autonome 2, dotée d'un interrupteur général marche/arrêt 3, et comportant une batterie 4 délivrant une tension constante de 7,2 volts.

Cette source d'alimentation 2 comporte, en outre, une prise extérieure 5 de connexion de la batterie 4 avec un chargeur, et un contrôleur de charge 6 apte à commander un interrupteur électronique 7 permettant de connecter la batterie 4, soit avec le réseau d'alimentation de l'appareil, soit avec la prise extérieure 5.

Cette source d'alimentation 2 comporte, enfin, des moyens 8 de détection de la charge de la batterie 4, et un voyant 9 disposé sur la face frontale du boîtier 1. Ce voyant 9 est connecté au contrôleur de charge 6 et aux moyens de détection 8 de façon :
- lorsqu'un chargeur est connecté à l'appareil : à être allumé lorsque l'appareil est en charge, et à s'éteindre lorsque la charge est terminée,
- lorsque l'appareil est utilisé : à être éteint lorsque la charge de la batterie 4 est correcte, et à clignoter lorsque cette batterie est à recharger.

L'appareil comprend, en second lieu, un générateur d'impulsions 10 doté de deux sorties sur chacune desquelles il délivre une impulsion rectangulaire positive d'une durée totale de T_{/2}, lesdites impulsions étant décalées de T_{/2}.

Ce générateur d'impulsions 10 est, en outre, adapté pour que la durée T soit de 300 µs ou de 600 µs, et il comprend un commutateur 11 disposé sur la face frontale du boîtier 1 pour la sélection manuelle de l'une ou l'autre durée.

L'appareil comporte, en outre, des moyens de temporisation connectés au générateur d'impulsions 10.

Ces moyens de temporisation comportent, en premier lieu, un oscillateur de base 12 apte à commander au générateur d'impulsions 10 de déclencher deux impulsions décalées de T_{/2}, soit toutes les 15 ms, soit toutes les 60 ms. Afin de permettre de sélectionner manuellement la périodicité, 15 ou 60 ms, cet oscillateur de base 12 comporte un interrupteur 13 disposé sur la face frontale du boîtier 1.

Les moyens de temporisation comprennent, en outre, une horloge 14 adaptée pour commander un arrêt de l'émission des impulsions après 8 mn de traitement, lorsque la périodicité de ces impulsions est de 15 ms.

Ces moyens de temporisation comprennent, enfin, un oscillateur de rapport de cycle 15 sollicité lorsque la durée des impulsions est de 15 ms et adapté pour scinder, de façon périodique, l'émission des impulsions en deux cycles : un cycle de travail dont la durée est de 3 s, au cours duquel les impulsions sont normalement délivrées toutes les 15 ms, et un cycle de repos dont la durée est de 9 s, au cours duquel l'émission des impulsions est stoppée.

L'appareil comprend, par ailleurs, deux circuits strictement identiques 16, 16' vers lesquels les impulsions sont délivrées en parallèle. Ces circuits sont dotés chacun d'une voie de sortie 17, 17' sur lesquelles sont générés des signaux identiques et en phases permettant un traitement simultané et synchrone de deux muscles à chronaxie identique d'un même patient.

Chacun de ces circuits 16, 16' comporte, en premier lieu, un générateur de courant constant 18, 18' alimenté par la batterie 4.

Ces circuits 16, 16' comportent, en outre, un transformateur 19, 19' doté de deux bobinages primaires 20a, 20b, 20'a, 20'b, inversés, possédant un point commun relié au générateur de courant 18, 18'. Ces transformateurs 19, 19' sont associés à des interrupteurs électroniques de commande 21, 22, 21', 22' reliant l'extrémité libre des bobinages primaires 20a, 20b, 20'a, 20'b à la masse de l'appareil, et agencés pour être activés chacun par une des impulsions délivrées par le générateur d'impulsions 10.

L'agencement de ces transformateurs 19, 19' permet de générer sur le bobinage secondaire 23, 23' et par conséquent de générer vers les voies 17, 17', un courant présentant la forme d'impulsions rectangulaires biphasiques à moyenne nulle et fronts raides dont la durée totale T est soit 300 µs, soit 600 µs.

Chaque circuit 16, 16' comporte, de plus, un potentiomètre 24, 24', disposé sur la face frontale du boîtier 1, adapté pour permettre d'obtenir un courant de sortie réglable de 0 à 100 mA sur chaque voie 17, 17'. Chacun de ces potentiomètres 24, 24' est, en outre, associé à un module 25, 25' de détection de sa position.

Les circuits 16, 16' sont, en outre, dotés d'une résistance 26, 26' de calibration du courant, interposée entre le générateur de courant 18, 18' et le transformateur 19, 19'.

Ces circuits comprennent, par ailleurs, des modules de sécurité aptes à interrompre, par action sur des interrupteurs électroniques 27, 28, 27', 28', l'émission des impulsions vers le transformateur 19, 19'.

Le premier de ces modules 29, 29' est adapté pour détecter la position des potentiomètres 24, 24' lors de la mise en marche de l'appareil, et pour commander l'ouverture des interrupteurs 27, 28, 27', 28' lorsque ces potentiomètres 24, 24' sont dans une position autre que la position "arrêt", c'est-à-dire une position différente de celle correspondant à un courant d'intensité nulle.

Le deuxième de ces modules 30 est commun aux deux circuits 16, 16' et est adapté pour détecter tout changement de programme en cours de traitement, c'est-à-dire toute action sur les commutateurs 11, 13 du générateur d'impulsions 10 et de l'oscillateur de base 12, et pour commander l'ouverture des interrupteurs 27, 28, 27', 28' lorsque ce changement de programme intervient alors que les potentiomètres 24, 24' ne sont pas dans leur position "arrêt".

Le troisième module 31, 31' est, quant à lui, adapté pour détecter des défauts, tels que surtension, sonde déconnectée, et pour commander l'ouverture des interrupteurs 27, 28, 27', 28' lors de l'apparition d'un tel défaut.

Chaque circuit 16, 16' comporte, enfin, un voyant 32, 32' disposé sur la face frontale du boîtier 1 et adapté pour indiquer l'état de la sortie 17, 17' correspondante de la façon suivante :
. voyant allumé : voie en fonctionnement,
. voyant éteint : voie éteinte (potentiomètre 24, 24' en position "arrêt"),
. voyant clignotant : défaut en sortie (surtension ou sonde déconnectée).

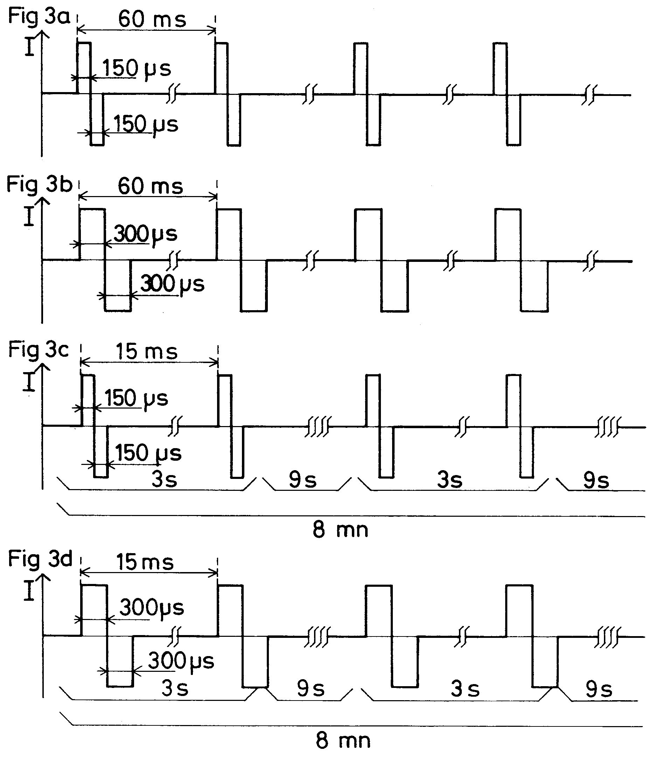

L'appareil ci-dessus décrit permet de générer quatre types de signaux identiques et en phase sur les deux voies 17, 17', qui sont représentés aux figures 3a à 3d.

Les deux premiers types de signaux représentés aux figures 3a et 3b se présentent sous la forme d'impulsions dont la durée totale est de 300 µs (figure 3a) ou de 600 µs (figure 3b), et dont la périodicité est de 60 ms. Sauf arrêt du traitement par l'utilisateur, ces impulsions sont délivrées sans interruption à partir de la mise en marche de l'appareil.

Les deux autres types de signaux représentés aux figures 3c et 3d se présentent sous la forme d'impulsions dont la durée totale est de 300 µs (figure 3c) ou de 600 µs (figure 3d), et dont la périodicité de 15 ms. En outre, la durée totale de traitement est limitée à 8 mn, et ce traitement comporte de façon alternative des cycles de travail d'une durée de 3 s, et des cycles de repos d'une durée de 9 s.

## Revendications

1. Appareil générateur de courant doté d'au moins une sortie de connexion (17) pour l'alimentation d'au moins deux sondes destinées à être appliquées sur une partie du corps humain en vue d'un traitement thérapeutique dans le domaine de la biophysique et de la physiologie, et comportant :
- une source (2) d'alimentation électrique autonome apte à délivrer une tension constante,
- des moyens (18) de génération d'un courant constant,
- des moyens (10, 19) générateurs de fréquence aptes à transformer le courant constant de façon à délivrer un courant présentant la forme d'impulsions rectangulaires biphasiques à moyenne nulle et fronts raides, dont la durée totale T en µs est sensiblement comprise entre 200 µs et 800 µs,
ledit appareil étant caractérisé en ce qu'il comprend des moyens de temporisation (12, 14, 15) de l'envoi des départs d'impulsions aptes à déclencher une impulsion toutes les k x 15 ms, avec k = 1 ou 4.

2. Appareil générateur de courant selon la revendication 1, caractérisé en ce que les moyens générateurs de fréquence (10, 19) sont adaptés pour délivrer des impulsions dont la durée totale T peut être soit 300 µs, soit 600 µs, et comprennent un commutateur (11) de sélection de l'une ou l'autre durée.

3. Appareil générateur de courant selon l'une des revendications 1 ou 2, caractérisé en ce que les moyens de temporisation comprennent :
- un oscillateur de base (12) doté d'un commutateur (13) apte à permettre de sélectionner la périodicité, 15 ms ou 60 ms, des impulsions,
- un oscillateur (15) de rapport de cycle agencé pour être actif lorsque la périodicité des impulsions est de 15 ms, et adapté pour scinder, de façon périodique, l'émission des impulsions en deux cycles de durées prédéterminées : un cycle de travail durant lequel les impulsions sont délivrées toutes les 15 ms, et un cycle de repos durant lequel aucune impulsion n'est délivrée.

4. Appareil générateur de courant selon la revendication 3, caractérisé en ce que l'oscillateur de rapport de cycle (15) est adapté pour que le rapport entre la durée du cycle de repos et la durée du cycle de travail soit de trois.

5. Appareil générateur de courant selon la revendication 4, caractérisé en ce que l'oscillateur de rapport de cycle (15) est adapté pour que la durée du cycle de repos soit de 9 s et celle du cycle de travail de 3 s.

6. Appareil générateur de courant selon l'une des revendications 3 à 5, caractérisé en ce que les moyens de temporisation comprennent une horloge (14) apte à interrompre l'émission des impulsions au bout d'un laps de temps prédéterminé après le début du traitement, lorsque la périodicité desdites impulsions est de 15 ms.

7. Appareil générateur de courant selon la revendication 6, caractérisé en ce que l'horloge (14) est programmée pour interrompre l'émission des impulsions au bout de 8 mn de traitement.

8. Appareil générateur de courant selon l'une des revendications précédentes, caractérisé en ce que les moyens générateurs de fréquence comprennent :
- un générateur d'impulsions (10) apte à délivrer des impulsions positives d'une durée totale de T_{/2}, décalées l'une par rapport à l'autre de T_{/2},
- au moins un transformateur (19) doté de deux bobinages primaires (20a, 20b) inversés possédant un point commun relié à un générateur de courant (18), lesdits bobinages primaires étant activés par le générateur d'impulsions (10) de façon à générer le courant rectangulaire biphasique sur le secondaire (23) du transformateur (19).

9. Appareil générateur de courant selon l'une des revendications précédentes, caractérisé en ce qu'il comprend des moyens (24) de réglage manuel de l'intensité du courant délivré vers chaque sortie de connexion (17), adaptés pour permettre de faire varier ladite intensité entre une valeur minimale nulle et une valeur prédéterminée.

10. Appareil générateur de courant selon la revendication 9, caractérisé en ce qu'il comprend des moyens (29) de détection de la position des moyens de réglage (24) de l'intensité du courant, aptes à interdire l'émission d'impulsions lorsque, lors de la mise en marche de l'appareil, lesdits moyens de réglage sont dans une position différente de celle correspondant à la valeur d'intensité nulle.

11. Appareil générateur de courant selon les revendications 2, 3 et 9 prises ensemble, caractérisé en ce qu'il comprend des moyens (30), dits de détection de changement de programme, aptes à interrompre l'émission d'impulsions lors de l'actionnement du commutateur (11, 13) de l'oscillateur de base (12) ou des moyens générateurs de fréquence (10), alors que les moyens de réglage (24) sont dans une position différente de celle correspondant à la valeur d'intensité nulle.

12. Appareil générateur de courant selon l'une des revendications précédentes, caractérisé en ce qu'il comprend des moyens (31) de détection de défauts, tels que surtension, sonde déconnectée, aptes à interdire l'émission d'impulsions lors de l'apparition d'un tel défaut.

13. Appareil générateur de courant selon l'une des revendications précédentes, caractérisé en ce que la source d'alimentation électrique (2) est constituée d'une batterie 7,2 V (4) associée à des moyens de contrôle de charge (6), des moyens (9) d'alarme de fin de charge, et à une prise extérieure (5) de connexion avec un chargeur.
